(19)

Europäisches Patentamt
European Patent Office
Office européen des brevets

(11) **EP 3 733 203 A1**

(12) **EUROPEAN PATENT APPLICATION**
published in accordance with Art. 153(4) EPC

(43) Date of publication:
**04.11.2020 Bulletin 2020/45**

(21) Application number: **18893830.2**

(22) Date of filing: **27.12.2018**

(51) Int Cl.:
*A61K 45/00* [(2006.01)]     *A61K 31/192* [(2006.01)]
*A61K 31/216* [(2006.01)]     *A61K 31/4178* [(2006.01)]
*A61P 3/10* [(2006.01)]     *A61P 27/12* [(2006.01)]
*A61P 43/00* [(2006.01)]

(86) International application number:
**PCT/JP2018/048211**

(87) International publication number:
**WO 2019/131897 (04.07.2019 Gazette 2019/27)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **28.12.2017 JP 2017254614**

(71) Applicants:
• **University of Fukui**
  **Fukui-shi, Fukui 910-8507 (JP)**
• **Santen Pharmaceutical Co., Ltd.**
  **Kita-ku**
  **Osaka-shi**
  **Osaka 530-8552 (JP)**

(72) Inventors:
• **OKI, Masaya**
  **Fukui-shi, Fukui 910-8507 (JP)**
• **KANADA, Fumito**
  **Fukui-shi, Fukui 910-8507 (JP)**

• **KAMATA, Kazuma**
  **Fukui-shi, Fukui 910-8507 (JP)**
• **TAKAMURA, Yoshihiro**
  **Yoshida-gun, Fukui 910-1193 (JP)**
• **MIYAKE, Seiji**
  **Yoshida-gun, Fukui 910-1193 (JP)**
• **INATANI, Masaru**
  **Yoshida-gun, Fukui 910-1193 (JP)**
• **UCHIDA, Hiroyuki**
  **Fukui-shi, Fukui 910-8507 (JP)**
• **NOGATA, Miho**
  **Fukui-shi, Fukui 910-8507 (JP)**
• **KATO, Masatomo**
  **Ikoma-shi, Nara 630-0101 (JP)**
• **FUJISAWA, Koushi**
  **Ikoma-shi, Nara 630-0101 (JP)**
• **TAKAOKA, Shinji**
  **Osaka-shi, Osaka 530-8552 (JP)**

(74) Representative: **Müller-Boré & Partner**
  **Patentanwälte PartG mbB**
  **Friedenheimer Brücke 21**
  **80639 München (DE)**

(54) **PROPHYLACTIC AGENT AND/OR THERAPEUTIC AGENT FOR CATARACT, MEDICINAL COMPOSITION FOR PREVENTING AND/OR TREATING CATARACT, USE OF PPAR ACTIVATOR FOR PRODUCING SAME, AND EYEDROPS**

(57)     Provided are agents for prevention and therapeutic treatment of cataract that act by a different mechanism from conventional agents, and use of a PPAR activator for production of such agents. An agent for prevention and/or therapeutic treatment of cataract, containing a PPAR activator as an active ingredient, is used.

FIG. 1

EP 3 733 203 A1

## Description

Technical Field

The present invention relates to an agent for prevention and/or therapeutic treatment of cataract, a pharmaceutical composition for prevention and/or therapeutic treatment of cataract, use of a PPAR activator for production of the agent or the composition, and an ophthalmic agent.

Background Art

Cataract is a disease that creates an opaque (e.g., milky) area in the crystalline lens and thereby causes decreased vision. Generally, when a person suffers from cataract, the opaque area occurs in the nucleus, cortex, posterior capsule, and/or the like of the crystalline lens.

Examples of the type of cataract include age-related cataract, diabetic cataract, and the like. Age-related cataract is the most prevalent type. For age-related cataract, the number of patients increases as the age increases. There is a report that about 66% to 85% of Japanese in their 60s, about 84% to 97% of Japanese in their 70s, and about 100% of Japanese in their 80s suffer from age-related cataract. On the contrary, diabetic cataract is prevalent among people in their 60s and younger. Diabetic cataract can also occur in the younger generation.

Cataract can be therapeutically treated, for example, by surgery or by administration of a preventive agent or a therapeutic agent. Examples of the preventive agent and therapeutic agent include agents that contain glutathione or pirenoxine (see Non-patent Literatures 1 and 2).

Citation List

[Non-patent Literature]

[Non-patent Literature 1]
Matensson. J et.al. (1989) Glutathione ester prevents buthionine sulfoximine-induced cataracts and lens epithelial cell damage, Proc Natl Acad Sci U S A, vol. 86, pp. 8727-8731.
[Non-patent Literature 2]
Ciuffi. M et. al. (1999) Protective Effect of Pirenoxine and U74389F on Induced Lipid Peroxidation in Mammalian Lenses. An in vitro, ex vivo and in vivo study, EXPERIMENTAL EYE RESEARCH, vol. 68, pp. 347-359.

Summary of Invention

Technical Problem

The foregoing conventional techniques, however, still have some room for improvement in the preventive effect and therapeutic effect on cataract. There is therefore a demand for a development of an agent for prevention and therapeutic treatment of cataract that acts by a different mechanism from conventional agents.

The present invention was made in view of the above conventional issue, and an object thereof is to provide an agent for prevention and/or therapeutic treatment of cataract that acts by a different mechanism from conventional agents, a pharmaceutical composition for prevention and/or therapeutic treatment of cataract that acts by a different mechanism from conventional agents, use of a PPAR activator for production of the agent or the composition, and a novel ophthalmic agent.

Solution to Problem

The inventors of the present invention have found that a PPAR activator (in other words, PPAR agonist) enables prevention and therapeutic treatment of cataract, and accomplished the present invention. Specifically, an embodiment of the present invention includes the following features.

<1> An agent for prevention and/or therapeutic treatment of cataract, containing a PPAR activator as an active ingredient.
<2> The agent described in <1>, in which the PPAR activator is at least one selected from the group consisting of fibrates, thiazolidines, glitazones, and glitazars.

<3> The agent described in <2>, in which the fibrates include ciprofibrate and gemfibrozil.

<4> The agent described in <2>, in which the thiazolidines include rosiglitazone.

<5> The agent described in any one of <1> to <4>, in which the agent is administered by instillation to an eye.

<6> The agent described in any one of <1> to <5>, in which the agent is an ophthalmic agent.

<7> The agent described in any one of <1> to <6>, in which the cataract is diabetic cataract.

<8> A pharmaceutical composition for prevention and/or therapeutic treatment of cataract, containing a PPAR activator as an active ingredient.

<9> The pharmaceutical composition described in <8>, in which the PPAR activator is at least one selected from the group consisting of fibrates, thiazolidines, glitazones, and glitazars.

<10> The pharmaceutical composition described in <9>, in which the fibrates include ciprofibrate and gemfibrozil.

<11> The pharmaceutical composition described in <9>, in which the thiazolidines include rosiglitazone.

<12> The pharmaceutical composition described in any one of <8> to <11>, in which the pharmaceutical composition is administered by instillation to an eye.

<13> The pharmaceutical composition described in any one of <8> to <12>, in which the pharmaceutical composition is an ophthalmic agent.

<14> The pharmaceutical composition described in any one of <8> to <13>, in which the cataract is diabetic cataract.

<15> Use of a PPAR activator for production of a medicament for prevention and/or therapeutic treatment of cataract.

<16> The use described in <15>, in which the PPAR activator is at least one selected from the group consisting of fibrates, thiazolidines, glitazones, and glitazars.

<17> The use described in <16>, in which the fibrates include ciprofibrate and gemfibrozil.

<18> The use described in <16>, in which the thiazolidines include rosiglitazone.

<19> The use described in any one of <15> to <18>, in which the medicament is administered by instillation to an eye.

<20> The use described in any one of <15> to <19>, in which the medicament is an ophthalmic agent.

<21> The use described in any one of <15> to <20>, in which the cataract is diabetic cataract.

<22> An ophthalmic agent containing ciprofibrate as an active ingredient.

<23> The ophthalmic agent described in <22>, in which the ciprofibrate is contained at a concentration of 0.001 to 10% (w/v).

<24> An ophthalmic agent containing gemfibrozil as an active ingredient.

<25> The ophthalmic agent described in <24>, in which the gemfibrozil is contained at a concentration of 0.001 to 10% (w/v).

<26> An ophthalmic agent containing rosiglitazone as an active ingredient.

<27> The ophthalmic agent described in <26>, in which the rosiglitazone is contained at a concentration of 0.001 to 10% (w/v).

<28> The ophthalmic agent described in any one of 22 to 27, in which the ophthalmic agent is for prevention and/or therapeutic treatment of cataract.

<29> The ophthalmic agent described in <28>, in which the cataract is diabetic cataract.

<30> The ophthalmic agent described in <28>, in which the cataract is age-related cataract.

An embodiment of the present invention also relates to the following features.

<31> A method for prevention and/or therapeutic treatment of cataract, including administering, to a subject (e.g., patient), a therapeutically effective amount of a PPAR activator.

<32> The method described in <31>, in which the PPAR activator is at least one selected from the group consisting of fibrates, thiazolidines, glitazones, and glitazars.

<33> The method described in <32>, in which the fibrates include ciprofibrate and gemfibrozil.

<34> The method described in <32>, in which the thiazolidines include rosiglitazone.

<35> The method described in any one of <31> to <34>, in which the PPAR activator is administered by instillation to an eye.

<36> The method described in any one of <31> to <35>, in which the PPAR activator is contained in an ophthalmic agent.

<37> The method described in any one of <31> to <36>, in which the cataract is diabetic cataract.

<38> A PPAR activator for use in prevention and/or therapeutic treatment of cataract.

<39> The PPAR activator described in <38>, in which the PPAR activator is at least one selected from the group consisting of fibrates, thiazolidines, glitazones, and glitazars.

<40> The PPAR activator described in <39>, in which the fibrates include ciprofibrate and gemfibrozil.

<41> The PPAR activator described in <39>, in which the thiazolidines include rosiglitazone.

<42> The PPAR activator described in any one of <38> to <41>, in which the PPAR activator is administered by instillation to an eye.

<43> The PPAR activator described in any one of <38> to <42>, in which the PPAR activator is contained in an ophthalmic agent.

<44> The PPAR activator described in any one of <38> to <43>, in which the cataract is diabetic cataract.

Advantageous Effects of Invention

According to an aspect of the present invention, it is possible to provide an agent for prevention and/or therapeutic treatment of cataract that acts by a different mechanism from conventional agents, a pharmaceutical composition for prevention and/or therapeutic treatment of cataract that acts by a different mechanism from conventional compositions, use of a PPAR activator for production of the agent or the composition, and a novel ophthalmic agent.

Brief Description of Drawings

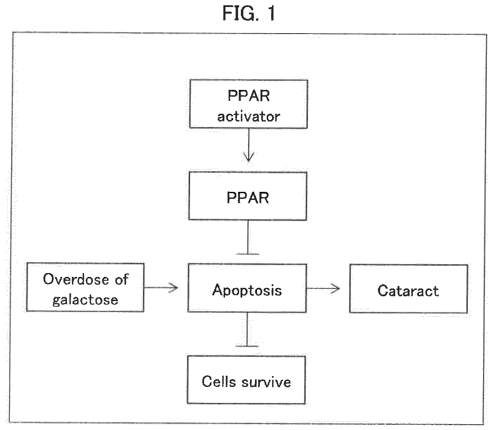

Fig. 1 is a model diagram showing an example of an action mechanism of an agent for prevention and/or therapeutic treatment of cataract in accordance with an embodiment of the present invention.

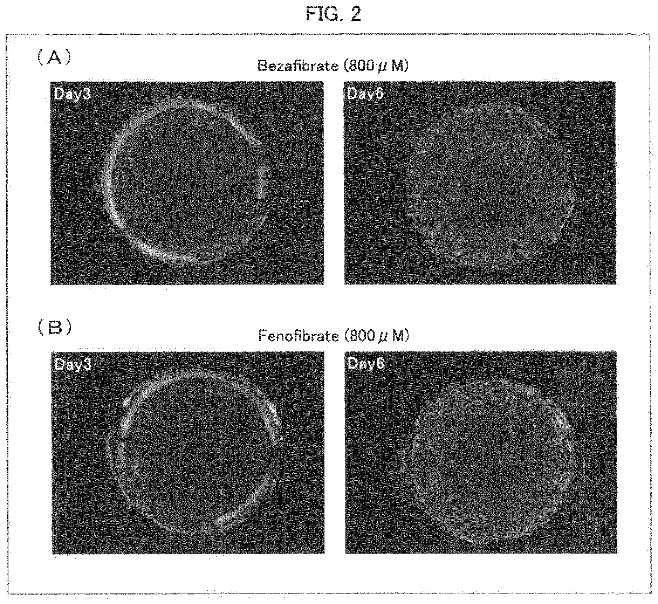

(A) and (B) of Fig. 2 show the effects, on cataract, of an agent for prevention and/or therapeutic treatment of cataract in accordance with an Example of the present invention.

Description of Embodiments

The following description will discuss an embodiment of the present invention. Note, however, that the present invention is not limited to the following embodiment. The present invention is not limited to the following arrangements, but can be altered by a skilled person in the art within the scope of the claims. The present invention also encompasses, in its technical scope, any embodiment and example derived by combining technical means disclosed in differing embodiments and/or examples. Furthermore, all of the literatures cited in this specification are incorporated herein by reference. In this specification, any numerical range represented in the form of "*A* to *B*" means "*A* or more and *B* or less".

[1. Putative action mechanism of the present invention]

The following describes a putative action mechanism of the present invention with reference to Fig. 1. Note that the mechanism discussed in this section is merely a putative action mechanism to help understand the present invention, and is not intended to limit the scope of the present invention.

The inventors of the present invention have conducted a study into cataract with use of ex-vivo diabetic cataract models using galactose-containing media. However, the inventors could not elucidate the mechanism by which galactose causes cataract or the mechanism by which galactose increases the severity of cataract.

During the study, the inventors have reached their own hypothesis that galactose may cause and/or increase the severity of cataract by the mechanism schematically shown in Fig. 1. Specifically, according to this mechanism, galactose induces apoptosis first, and the apoptosis causes and/or increases the severity of cataract.

The inventors have further made their own hypothesis that a peroxisome proliferator-activated receptor (PPAR) plays an important role with regard to apoptosis in an aspect in which the apoptosis causes and/or increases the severity of cataract, and demonstrated that cataract can be prevented and therapeutically treated by a PPAR activator as described later in Examples. The inventors thus accomplished the present invention.

[2. Definition of terms]

[2-1. Cataract]

In this specification, the term "cataract" refers to a disease that creates an opaque (e.g., milky) area in the crystalline lens. Examples of the type of opacity that is caused by cataract in the crystalline lens include: cortical opacity (CO); nuclear opacity (NO); posterior subcapsular opacity (PSO); cortical spoke (CS); anterior subcapsular opacity (ASC); fiber folds (FF), waterclefts (WC), and perinuclear retrodots (RD); vacuoles (VC); focal dots (FD); and coronary flakes (CF). The meaning of the term "cataract" as used herein includes, for example, age-related cataract (senile cataract), congenital cataract, and complicated cataract (e.g., diabetic cataract, traumatic cataract, atopic cataract, radiation cataract, and steroid cataract).

The term "diabetic cataract" as used herein refers to opacity of the crystalline lens developed in a patient of a disease characterized by continuous high glucose levels in blood or blood plasma (such a disease is, for example, hyperglycemia or diabetes (e.g., type I diabetes or type II diabetes)). A large-scale epidemiologic survey has revealed that diabetic cataract causes opacity often in the cortex or under the posterior capsule of the crystalline lens; however, opacity may occur in areas other than these areas.

[2-2. Preventive agent]

The term "preventive agent" as used herein refers to an agent that provides a preventive effect. The term "preventive effect" refers to, but is not limited to, any one or more of the effects listed below as examples.

(1) Administration of the preventive agent prevents development of one or more symptoms of a disease or reduces the risk of development of one or more symptoms of the disease, as compared to cases in which no preventive agents are administered.
(2) Administration of the preventive agent prevents relapse of one or more symptoms of a disease or reduces the risk of relapse of one or more symptoms of the disease, as compared to cases in which no preventive agents are administered.
(3) Administration of the preventive agent prevents signs of one or more symptoms of a disease from occurring or reduces the risk of occurrence of signs of one or more symptoms of the disease, as compared to cases in which no preventive agents are administered.

Note that one or more symptoms of a disease may be systemic or local.

[2-3. Therapeutic agent]

The term "therapeutic agent" as used herein refers to an agent that provides a therapeutic effect. The term "therapeutic effect" refers to, but is not limited to, any one or more of the effects listed below as examples.

(1) Administration of the therapeutic agent reduces severity of one or more symptoms of a disease, as compared to cases in which no therapeutic agents are administered.
(2) Administration of the therapeutic agent prevents severity of one or more symptoms of a disease from increasing or prevents one or more symptoms of the disease from progressing, as compared to cases in which no therapeutic agents are administered.
(3) Administration of the therapeutic agent reduces the rate of increase of severity of one or more symptoms of a disease or rate of progress of one or more symptoms of the disease, as compared to cases in which no therapeutic agents are administered.

Note that one or more symptoms of a disease may be systemic or local.
It should be noted here that the term "agent for prevention and/or therapeutic treatment" refers to an agent that serves as at least one of the preventive agent and the therapeutic agent and that provides any of the above-listed effects. The "agent for prevention and/or therapeutic treatment" can be reworded as "treatment agent". Similarly, the term "pharmaceutical composition for prevention and/or therapeutic treatment" can be reworded as "pharmaceutical composition for treatment", the term "medicament for prevention and/or therapeutic treatment" can be reworded as "medicament for treatment", the term "method for prevention and/or therapeutic treatment" can be reworded as "method of treatment", and the term "use in prevention and/or therapeutic treatment" can be reworded as "use in treatment".

[3. PPAR activator]

A PPAR activator (in other words, PPAR agonist) is a substance that activates a peroxisome proliferator-activated receptor (PPAR) and upregulates PPAR signaling pathway. In other words, the PPAR activator is a compound that binds to a PPAR and thereby provides an agonist effect. The PPAR activator is also called "PPAR agonist". There are subtypes of PPAR (specifically, PPAR-$\alpha$, PPAR-$\beta$, PPAR-$\gamma$, and PPAR-$\delta$). A PPAR activated by a PPAR activator in accordance with the present embodiment may be of any subtype.
The PPAR activator in accordance with the present embodiment may be PPAR-$\alpha$ activator, PPAR-$\beta$ activator, PPAR-$\gamma$ activator, or PPAR-$\delta$ activator. Alternatively, the PPAR activator in accordance with the present embodiment may be a PPAR dual agonist (dual PPAR activator which activates a plurality of PPAR subtypes concurrently) or a pan-PPAR activator (PPAR activator that has low specificity and that activates PPAR subtypes concurrently in a uniform manner). The agonist effect may be full agonist effect or partial agonist effect.
The PPAR activator in accordance with the present embodiment may be a fibrate, a thiazolidine, a glitazone, a glitazar, propionic acid, a terpene (e.g., monoterpene, sesquiterpene, diterpene, triterpene, steroid, or carotenoid), a polyketide (e.g., anthraquinone or prenylated polyketide), a phenylpropanoid (e.g., coumarin, lignan, or tannin), a polyphenol (e.g., chalcone, stilbene, flavonoid, isoflavonoid, or biflavonoid), or an alkaloid. It is more preferable that the PPAR activator in accordance with the present embodiment is at least one selected from the group consisting of fibrates, thiazolidines, glitazones, and glitazars. It is even more preferable that the PPAR activator in accordance with the present embodiment is at least one selected from the group consisting of fibrates and thiazolidines.

The following is a list of examples of a substance that can serve as the PPAR activator in accordance with the present embodiment. Note, however, that the present invention is not limited to such substances. Note that, in the following list, each substance's data consists of "(a) Name of the substance", "(b) IUPAC name of the substance", "(c) PPAR subtype targeted by the substance", "(d) Classification 1 of the substance", and "(e) Classification 2 of the substance". Also note that, in the following list, the symbol "-" means that there are no appropriate data or that the substance is difficult to classify.

[1] (a) Bezafibrate; (b) 2-[4-[2-[(4-chlorobenzoyl)amino]ethyl]phenoxy]-2-methylpropanoic acid; (c) $\alpha$, $\delta$, $\gamma$; (d) -; (e) fibrate.

[2] (a) Fenofibrate; (b) propan-2-yl 2-[4-(4-chlorobenzoyl)phenoxy]-2-methylpropanoate; (c) $\alpha$; (d) -; (e) fibrate.

[3] (a) Ciprofibrate; (b) 2-[4-(2,2-dichlorocyclopropyl)phenoxy]-2-methylpropanoic acid; (c) $\alpha$; (d) -; (e) fibrate.

[4] (a) Clofibrate; (b) ethyl 2-(4-chlorophenoxy)-2-methylpropanoate; (c) $\alpha$; (d) -; (e) fibrate.

[5] (a) GW7647; (b) 2-[4-[2-[4-cyclohexylbutyl(cyclohexylcarbamoyl)amino]ethyl]phenyl]su lfanyl-2-methylpropanoic acid; (c) $\alpha$; (d) -; (e) -.

[6] (a) Leukotriene B$_4$; (b) (5S,6Z,8E,10E,12R,14Z)-5,12-dihydroxyicosa-6,8,10,14-tetraenoic acid; (c) $\alpha$; (d) -; (e) -.

[7] (a) Oleylethanolamide; (b) (Z)-N-(2-hydroxyethyl)octadec-9-enamide; (c) $\alpha$; (d) -; (e) -.

[8] (a) Tetradecylthioacetic Acid; (b) 2-tetradecylsulfanylacetic acid; (c) $\alpha$; (d) -; (e) -.

[9] (a) WY-14643; (b) 2-[4-chloro-6-(2,3-dimethylanilino)pyrimidin-2-yl]sulfanylacetic acid; (c) $\alpha$; (d) -; (e) -.

[10] (a) GW0742; (b) 2-[4-[[2-[3-fluoro-4-(trifluoromethyl)phenyl]-4-methyl-1,3-thiazol-5-yl]methylsulfanyl]-2-methyl-phenoxy]acetic acid; (c) $\delta$;(d) -; (e) -.

[11] (a) L-165041; (b) 2-[4-[3-(4-acetyl-3-hydroxy-2-propylphenoxy)propoxy]phenoxy]acetic acid; (c) $\beta$,$\delta$, $\gamma$; (d) -; (e) -.

[12] (a) Ciglitazone; (b) 5-[[4-[(1-methylcyclohexyl)methoxy]phenyl]methyl]-1,3-thiazolidine-2,4-dione; (c) $\gamma$; (d) Glitazone; (e) Thiazolidinedione.

[13] (a) GW1929; (b) (2S)-2-(2-benzoylanilino)-3-[4-[2-[methyl(pyridin-2-yl)amino]ethoxy]phenyl]propanoic acid; (c) $\gamma$; (d) -; (e) -.

[14] (a) nTZDpa; (b) 5-chloro-1-[(4-chlorophenyl)methyl]-3-phenylsulfanylindole-2-carboxylic acid; (c) $\gamma$; (d) -; (e) -.

[15] (a) Pioglitazone Hydrochloride; (b) 5-[[4-[2-(5-ethylpyridin-2-yl)ethoxy]phenyl]methyl]-1,3-thiazolidine-2,4-di-one;hydrochloride; (c) $\gamma$; (d) Glitazone; (e) Thiazolidinedione.

[16] (a) Rosiglitazone; (b) 5-[[4-[2-[methyl(pyridin-2-yl)amino]ethoxy]phenyl]methyl]-1,3-thiazolidine-2,4-dione; (c) $\gamma$; (d) Glitazone; (e) Thiazolidinedione.

[17] (a) TIPP-703; (b) (2S)-2-[[3-[[[4-(1-adamantyl)benzoyl]amino]methyl]-4-propoxyphenyl]methyl]butanoic acid; (c) $\gamma$; (d) -; (e) -.

[18] (a) Troglitazone; (b) 5-[[4-[(6-hydroxy-2,5,7,8-tetramethyl-3,4-dihydrochromen-2-yl)methoxy]phenyl]methyl]-1,3-thiazolidine-2,4-dione; (c) $\gamma$; (d) Glitazone; (e) Thiazolidinedione.

[19] (a) Pioglitazone; (b) 5-[[4-[2-(5-ethylpyridin-2-yl)ethoxy]phenyl]methyl]-1,3-thiazolidine-2,4-dione; (c) $\gamma$; (d) Glitazone; (e) Thiazolidinedione.

[20] (a) Telmisartan; (b) 2-[4-[[4-methyl-6-(1-methylbenzimidazol-2-yl)-2-propylbenzimidazol-1-yl]methyl]phe-nyl]benzoic acid; (c) $\delta$, $\gamma$; (d) -; (e) -.

[21] (a) Gemfibrozil; (b) 5-(2,5-dimethylphenoxy)-2,2-dimethylpentanoicacid; (c) $\alpha$; (d) -; (e) fibrate.

[22] (a) Palmitoylethanolamide; (b) N-(2-hydroxyethyl)hexadecanamide; (c) $\alpha$; (d) -; (e) -.

[23] (a) Farglitazar; (b) (2S)-2-(2-benzoylanilino)-3-[4-[2-(5-methyl-2-phenyl-1,3-oxazol-4-yl)ethoxy]phenyl]propa-noic acid; (c) $\alpha$, $\gamma$; (d) glitazar; (e) -.

[24] (a) Tesaglitazar; (b) (2S)-2-ethoxy-3-[4-[2-(4-methylsulfonyloxyphenyl)ethoxy]phenyl]propanoic acid; (c) $\alpha$, $\gamma$; (d) glitazar; (e) Non-thiazolidinedione type PPAR agonist.

[25] (a) Muraglitazar (BMS-298585); (b)2-[(4-methoxyphenoxy)carbonyl-[[4-[2-(5-methyl-2-phenyl-1,3-oxazol-4-yl)ethoxy]phenyl]methyl]amino]acetic acid; (c) $\alpha$, $\gamma$; (d) glitazar; (e) Non-thiazolidinedione type PPAR agonist.

[26] (a) INT-131; (b) 2,4-dichloro-N-(3,5-dichloro-4-quinolin-3-yloxyphenyl)benzenesulfonamide; (c) $\gamma$; (d) -; (e)

[27] (a) MK-0533; (b) (2R)-2-[3-[3-(4-methoxybenzoyl)-2-methyl-6-(trifluoromethoxy)indol-1-yl]phenoxy]butanoic acid; (c) $\gamma$; (d) -; (e) -.

[28] (a) Aleglitazar; (b) (2S)-2-methoxy-3-[4-[2-(5-methyl-2-phenyl-1,3-oxazol-4-yl)ethoxy]-1-benzothiophen-7-yl]propanoic acid; (c) $\alpha$, $\gamma$; (d) glitazar; (e) -.

[29] (a) Elafibranor (GFT505); (b) 2-[2,6-dimethyl-4-[(E)-3-(4-methylsulfanylphenyl)-3-oxoprop-1-enyl]phenoxy]-2-methylpropanoic acid; (c) $\alpha$, $\delta$; (d) -; (e) -.

[30] (a) Saroglitazar; (b) (2S)-2-ethoxy-3-[4-[2-[2-methyl-5-(4-methylsulfanylphenyl)pyrrol-1-yl]ethoxy]phenyl]propa-noic acid; (c) $\alpha$, $\gamma$; (d) glitazar; (e) -.

[31] (a) Chiglitazar; (b) (2S)-3-[4-(2-carbazol-9-ylethoxy)phenyl]-2-[2-(4-fluorobenzoyl)anilino]propanoic acid; (c) $\alpha$,

γ; (d) glitazar; (e) -.

[32] (a) GW 9578; (b) 2-[4-[2-[(2,4-difluorophenyl)carbamoyl-heptylamino]ethyl]phenyl]sulfanyl-2-methylpropanoic acid; (c) α; (d) -; (e) -.

[33] (a) LY 518674; (b) 2-methyl-2-[4-[3-[2-[(4-methylphenyl) methyl]-3-oxo-1H-1,2,4-triazol-5-yl]propyl]phenoxy]propanoic acid; (c) α; (d) -; (e) -.

[34] (a) GW 501516; (b) 2-[2-methyl-4-[[4-methyl-2-[4-(trifluoromethyl)phenyl]-1,3-thiazol-5-yl]methylsulfanyl]phenoxy]acetic acid; (c) β, γ; (d) -; (e)-.

[35] (a) Retinoic acid; (b) (2E,4E,6E,8E)-3,7-dimethyl-9-(2,6,6-trimethylcyclohexen-1-yl)nona-2,4,6,8-tetraenoic acid; (c) β, γ; (d) -; (e) -.

[36] (a) Indomethacin; (b) 2-[1-(4-chlorobenzoyl)-5-methoxy-2-methylindol-3-yl]acetic acid; (c) γ; (d) -; (e) -.

[37] (a) Fenoprofen; (b)2-(3-phenoxyphenyl)propanoic acid; (c) γ; (d) propionic acid; (e) -.

[38] (a) Ibuprofen; (b)2-[4-(2-methylpropyl)phenyl]propanoic acid; (c) γ; (d) propionic acid; (e) -.

[39] (a) KRP-297; (b) 5-[(2,4-dioxo-1,3-thiazolidin-5-yl)methyl]-2-methoxy-N-[[4-(trifluoromethyl)phenyl]methyl]benzamide; (c) γ; (d) -; (e) -.

[40] (a) Ragaglitazar; (b) (2S)-2-ethoxy-3-[4-(2-phenoxazin-10-ylethoxy)phenyl]propanoic acid; (c) α, γ; (d) glitazar; (e) -.

[41] (a) LY 929 (LY-510929); (b) (2S)-2-methyl-3-[4-[2-(5-methyl-2-thiophen-2-yl-1,3-oxazol-4-yl)ethoxy]phenyl]-2-phenoxypropanoic acid; (c) α, γ; (d) -; (e) -.

[42] (a) LSN862; (b) (S)-2-methoxy-3-{4-[5-(4-phenoxy)pent-1-ynyl]phenyl}propionic acid; (c) α, γ; (d) -; (e) -.

[43] (a) Sodelglitazar (GW677954); (b) 2-[4-[[2-[2-fluoro-4-(trifluoromethyl)phenyl]-4-methyl-1,3-thiazol-5-yl]methylsulfanyl]-2-methylphenoxy]-2-methylpropanoic acid; (c) α, γ, δ; (d) glitazar; (e) -.

[44] (a) Indeglitazar (PLX204); (b) 3-[5-methoxy-1-(4-methoxyphenyl)sulfonylindol-3-yl]propanoic acid; (c) α, γ, δ; (d) glitazar; (e) -.

[45] (a) DRL 11605; (b) -; (c) α, γ, δ; (d) -; (e) -.

[46] (a) Linoleic acid; (b) (9Z,12Z)-octadeca-9,12-dienoic acid; (c) α; (d) -; (e) -.

[47] (a) Arachidonic acid; (b) (5Z,8Z,11Z,14Z)-icosa-5,8,11,14-tetraenoic acid; (c) α; (d) -; (e) -.

[48] (a) 8-S-hydroxytetraenoic acid (8-S-HETE); (b) (5Z,8S,9E,11Z,14Z)-8-hydroxyicosa-5,9,11,14-tetraenoic acid; (c) α; (d) -; (e) -.

[49] (a) Carbaprostacyclin; (b) (5E)-5-[(3aS,4R,5R,6aS)-5-hydroxy-4-[(E,3S)-3-hydroxyoct-1-enyl]-3,3a,4,5,6,6a-hexahydro-1H-pentalen-2-ylidene]pentanoic acid; (c) β, γ; (d) -; (e) -.

[50] (a) Prostaglandin J2; (b) (Z)-7-[(1S,5R)-5-[(E,3S)-3-hydroxyoct-1-enyl]-4-oxocyclopent-2-en-1-yl]hept-5-enoic acid; (c) γ; (d) -; (e) -.

[51] (a) 15-Deoxy-Δ12,14-prostaglandin J2; (b) 1,3-dihydroxypropan-2-yl (Z)-7-[(1S,5E)-5-[(Z)-oct-2-enylidene]-4-oxocyclopent-2-en-1-yl]hept-5-enoate; (c) γ; (d) -; (e) -.

[52] (a) 15-Hydroxytetraenoic acid (15-HETE); (b) (5E,8E,11E,13E)-15-hydroxyicosa-5,8,11,13-tetraenoic acid; (c) γ; (d) -; (e) -.

[53] (a) Prostacyclin; (b) (5Z)-5-[(3aR,4R,5R,6aS)-5-hydroxy-4-[(E,3S)-3-hydroxyoct-1-enyl]-3,3a,4,5,6,6a-hexahydrocyclopenta[b]furan-2-ylidene]pentanoic acid; (c) β, γ; (d) -; (e) -.

[54] (a) Pemafibrate (K-877); (b) (2R)-2-[3-[[1,3-benzoxazol-2-yl-[3-(4-methoxyphenoxy)propyl]amino]methyl]phenoxy]butanoic acid; (c)α; (d) -; (e) fibrate.

[55] (a) thiazolidinedione; (b) 1,3-thiazolidine-2,4-dione; (c) γ; (d) -; (e) -.

[56] (a) Honokiol; (b) 2-(4-hydroxy-3-prop-2-enylphenyl)-4-prop-2-enylphenol; (c) γ; (d) -; (e) -.

[57] (a) amorfrutin; (b) 3-[(2E)-3,7-dimethylocta-2,6-dienyl]-2-hydroxy-4-methoxy-6-(2-phenylethyl)benzoic acid; (c) γ; (d) -; (e) -.

[58] (a) amorphastilbol; (b) 2-[(2E)-3,7-dimethylocta-2,6-dienyl]-5-[(E)-2-phenylethenyl]benzene-1,3-diol; (c) γ; (d) -; (e) -.

[59] (a) linalool; (b) 3,7-dimethylocta-1,6-dien-3-ol; (c) α; (d) Monoterpenes; (e) Terpenes.

[60] (a) carvacrol; (b) 2-methyl-5-propan-2-ylphenol; (c) α; (d) Monoterpenes; (e) Terpenes.

[61] (a) thymol; (b) 5-methyl-2-propan-2-ylphenol; (c) α; (d) Monoterpenes; (e) Terpenes.

[62] (a) excelside B; (b) methyl (4S,5E)-5-ethylidene-4-[2-[2-(4-hydroxyphenyl)ethoxy]-2-oxoethyl]-6-[(2S,3R,4S,5S,6R)-3,4,5-trihydroxy-6-[[(2R,3R,4S,5S,6R)-3,4,5-trihydroxy-6-(hydroxymethyl)oxan-2-yl]oxymethyl]oxan-2-yl]oxy-4H-pyran-3-carboxylate; (c) α; (d) Monoterpenes; (e) Terpenes.

[63] (a) trans-Caryophyllene; (b) (4E)-4,11,11-trimethyl-8-methylidenebicyclo[7.2.0]undec-4-ene; (c) α; (d Sesquiterpenes; (e) Terpenes.

[64] (a) farnesol; (b) (2E,6E)-3,7,11-trimethyldodeca-2,6,10-trien-1-ol; (c) α; (d) Sesquiterpenes; (e) Terpenes.

[65] (a) dehydroabietic acid; (b) (1R,4aS,10aR)-1,4a-dimethyl-7-propan-2-yl-2,3,4,9,10,10a-hexahydrophenanthrene-1-carboxylic acid; (c) α; (d) Diterpenes; (e) Terpenes.

[66] (a) (E)-phytol; (b) (E,7R,11R)-3,7,11,15-tetramethylhexadec-2-en-1-ol; (c) α; (d) Diterpenes; (e) Terpenes.

[67] (a) phytanic acid; (b) 3,7,11,15-tetramethylhexadecanoic acid; (c) $\alpha$; (d) Diterpenes; (e) Terpenes.

[68] (a) geranylgeraniol; (b) (2E,6E,10E)-3,7,11,15-tetramethylhexadeca-2,6,10,14-tetraen-1-ol; (c) $\alpha$, $\gamma$; (d) Diterpenes; (e) Terpenes.

[69] (a) Pseudolaric acid B; (b) (2E,4E)-5-[(1R,7S,8R,9R)-4,7-Bis(methoxycarbonyl)-9-methyl-11-oxo-10-oxatricyclo[6.3.2.0$^{1.7}$]tridec-3-en-9-yl]-2-methyl-2,4-pentadienoic acid; (c) $\alpha$, $\beta$, $\gamma$; (d) Diterpenes; (e) Terpenes.

[70] (a) Oleanic acid; (b) (4aS,6aR,6aS,6bR,8aR,10S,12aR,14bS)-10-hydroxy-2,2,6a,6b,9,9,12a-heptamethyl-1,3,4,5,6,6a, 7,8,8a, 10,11,12,13,14b-tetradecahydropicene-4a-carboxylic acid; (c) $\alpha$; (d) Triterpenes; (e) Terpenes.

[71] (a) ginsenoside Rf; (b) (2S,3R,4S,5S,6R)-2-[(2R,3R,4S,5S,6R)-2-[[(3S,5R,6S,8R,9R,10R,12R,13R,14R,17S)-3,12-dihydroxy-17-[(2S)-2-hydroxy-6-methylhept-5-en-2-yl]-4,4,8,10,14-pentamethyl-2,3,5,6,7,9,11,12,13,15,16,17-dodecahydro-1H-cyclopenta[a]phenanthren-6-yl]oxy]-4,5-dihydroxy-6-(hydroxymethyl)oxan-3-yl]oxy-6-(hydroxymethyl)oxane-3,4,5-triol; (c) $\alpha$; (d) steroids; (e) Terpenes.

[72] (a) Fucoxanthin; (b) [(1S,3R)-3-hydroxy-4-[(3E,5E,7E,9E,11E,13E,15E)-18-[(1R,3S,6S)-3-hydroxy-1,5,5-trimethyl-7-oxabicyclo[4.1.0]heptan-6-yl]-3,7,12,16-tetramethyl-17-oxooctadeca-1,3,5,7,9,11,13,15-octaenylidene]-3,5,5-trimethylcyclohexyl] acetate; (c) $\alpha$; (d) Carotenoids; (e) Terpenes.

[73] (a) Sargaquinoic acid; (b) (2E,6E,10E)-6,10-dimethyl-12-(5-methyl-3,6-dioxocyclohexa-1,4-dien-1-yl)-2-(4-methylpent-3-enyl)dodeca-2,6,10-trienoic acid; (c) $\alpha$; (d) Carotenoids; (e) Terpenes.

[74] (a) sargahydroquinoic acid; (b) (2Z,6E,10E)-12-(2,5-dihydroxy-3-methylphenyl)-6,10-dimethyl-2-(4-methylpent-3-enyl)dodeca-2,6,10-trienoic acid; (c) $\alpha$; (d) Carotenoids; (e) Terpenes.

[75] (a) Bixin; (b) (2E,4E,6E,8E,10E,12E,14E,16E,18E)-20-methoxy-4,8,13,17-tetramethyl-20-oxoicosa-2,4,6,8,10,12,14,16,18-nonaenoic acid; (c) $\alpha$; (d) Carotenoids; (e) Terpenes.

[76] (a) monotriajaponide A; (b) (2E,4E,9E)-4,6,8-triethyldodeca-2,4,9-trienoic acid; (c) $\alpha$, $\gamma$; (d) Polyketides; (e) Polyketides.

[77] (a) mangiferin; (b) 1,3,6,7-tetrahydroxy-2-[(2S,3R,4R,5S,6R)-3,4,5-trihydroxy-6-(hydroxymethyl)oxan-2-yl]xanthen-9-one; (c) $\alpha$, $\gamma$; (d) Anthraquinones; (e) Polyketides.

[78] (a) norathyriol; (b) 1,3,6,7-tetrahydroxyxanthen-9-one; (c) $\alpha$; (d) Anthraquinones; (e) Polyketides.

[79] (a) Isohumulone; (b) 3,4-dihydroxy-2-(3-methylbutanoyl)-5-(3-methylbut-2-enyl)-4-(4-methylpent-3-enoyl)cyclopent-2-en-1-one; (c) $\alpha$, $\gamma$; (d) Prenylatedpolyketides; (e) Polyketides.

[80] (a) isocohumulone; (b) (4R,5S)-3,4-dihydroxy-5-(3-methylbut-2-enyl)-4-(4-methylpent-3-enoyl)-2-(2-methylpropanoyl)cyclopent-2-en-1-one; (c) $\alpha$, $\gamma$; (d) Prenylatedpolyketides; (e) Polyketides.

[81] (a) Rosmarinic acid; (b) (2R)-3-(3,4-dihydroxyphenyl)-2-[(E)-3-(3,4-dihydroxyphenyl)prop-2-enoyl]oxypropanoic acid; (c) $\alpha$; (d) Phenylpropanoids; (e) Phenylpropanoids.

[82] (a) Acteoside; (b) [(2R,3R,4R,5R,6R)-6-[2-(3,4-dihydroxyphenyl)ethoxy]-5-hydroxy-2-(hydroxymethyl)-4-[(2S,3R,4R,5R,6S)-3,4,5-trihydroxy-6-methyloxan-2-yl]oxyoxan-3-yl](E)-3-(3,4-dihydroxyphenyl)prop-2-enoate; (c) $\alpha$; (d) Phenylpropanoids; (e) Phenylpropanoids.

[83] (a) umbelliferone; (b) 7-hydroxychromen-2-one; (c) $\alpha$; (d) Coumarins; (e) Phenylpropanoids.

[84] (a) osthole; (b) 7-methoxy-8-(3-methylbut-2-enyl)chromen-2-one; (c) $\alpha$, $\gamma$; (d) Coumarins; (e) Phenylpropanoids.

[85] (a) auraptene; (b) 7-[(2E)-3,7-dimethylocta-2,6-dienoxy]chromen-2-one; (c) $\alpha$, $\gamma$; (d) Coumarins; (e) Phenylpropanoids.

[86] (a) Sesamin; (b) 5-[(3S,3aR,6S,6aR)-3-(1,3-benzodioxol-5-yl)-1,3,3a,4,6,6a-hexahydrofuro[3,4-c]furan-6-yl]-1,3-benzodioxole; (c) $\alpha$; (d) Lignans; (e) Phenylpropanoids.

[87] (a) sesamol; (b) 1,3-benzodioxol-5-ol; (c) $\alpha$; (d) Lignans; (e) Phenylpropanoids.

[88] (a) corilagin; (b) [3,5-dihydroxy-2-(3,4,5-trihydroxybenzoyl)oxy-6-[(3,4,5-trihydroxybenzoyl)oxymethyl]oxan-4-yl] 3,4,5-trihydroxybenzoat; (c) $\alpha$, $\gamma$; (d) Tannins; (e) Phenylpropanoids.

[89] (a) 1,2,3,4,6-penta-O-galloyl-$\beta$-d-glucose; (b) [(2R,3R,4S,5R,6S)-3,4,5,6-tetrakis[(3,4,5-trihydroxybenzoyl)oxy]oxan-2-yl]methyl 3,4,5-trihydroxybenzoate; (c) $\alpha$, $\gamma$; (d) Tannins; (e) Phenylpropanoids.

[90] (a) panduratin A; (b) (2,6-dihydroxy-4-methoxyphenyl)-[(1R,2S,6R)-3-methyl-2-(3-methylbut-2-enyl)-6-phenylcyclohex-3-en-1-yl]methanone; (c) $\alpha$, $\delta$; (d) chalcone; (e) Polyphenols.

[91] (a) Resveratrol; (b) 5-[(E)-2-(4-hydroxyphenyl)ethenyl]benzene-1,3-diol; (c) $\alpha$, $\gamma$; (d) stilbene; (e) Polyphenols.

[92] (a) pterostilbene; (b) 4-[(E)-2-(3,5-dimethoxyphenyl)ethenyl]phenol; (c) $\alpha$; (d) stilbene; (e) Polyphenols.

[93] (a) Vaticanol C; (b) (1R,2R,3R,4R,10S,11R)-4-(3,5-Dihydroxyphenyl)-3,11-bis(4-hydroxyphenyl)hexacyclo[8.7.6.1$^{2,5}$.0$^{9,24}$.0$^{12,17}$.0$^{18,23}$]tetraco sa-5(24),6,8,12,14,16,18,20,22-nonaene-6,8,14,19,21-pentol; (c) $\alpha$, $\beta$, $\delta$; (d) stilbene; (e) Polyphenols.

[94] (a) hesperetin; (b) (2S)-5,7-dihydroxy-2-(3-hydroxy-4-methoxyphenyl)-2,3-dihydrochromen-4-one; (c) $\gamma$; (d) Flavonoids; (e) Polyphenols.

[95] (a) naringenin; (b) 5,7-dihydroxy-2-(4-hydroxyphenyl)-2,3-dihydrochromen-4-one; (c) $\alpha$, $\gamma$; (d) Flavonoids; (e) Polyphenols.

[96] (a) Hispidulin; (b) 5,7-dihydroxy-2-(4-hydroxyphenyl)-6-methoxychromen-4-one; (c) $\alpha$; (d) Flavonoids; (e) Polyphenols.

[97] (a) 5,7-dimethoxyflavone; (b) 5,7-dimethoxy-2-phenylchromen-4-one; (c) α, γ; (d) Flavonoids; (e) Polyphenols.

[98] (a) Icariin; (b) 5-hydroxy-2-(4-methoxyphenyl)-8-(3-methylbut-2-enyl)-7-[(2S,3R,4S,5S,6R)-3,4,5-trihydroxy-6-(hydroxymethyl)oxan-2-yl]oxy-3-[(2S,3R,4R,5R,6S)-3,4,5-trihydroxy-6-methyloxan-2-yl]oxychromen-4-one; (c) α, γ; (d) Flavonoids; (e) Polyphenols.

[99] (a) Epigallocatechin-3-gallate; (b) [(2R,3R)-5,7-dihydroxy-2-(3,4,5-trihydroxyphenyl)-3,4-dihydro-2H-chromen-3-yl] 3,4,5-trihydroxybenzoate; (c) α; (d) Flavonoids; (e) Polyphenols.

[100] (a) genistein; (b) 5,7-dihydroxy-3-(4-hydroxyphenyl)chromen-4-one; (c) α; (d) Isoflavonoids; (e) Polyphenols.

[101] (a) 3'-hydroxygenistein; (b) 3-(3,4-dihydroxyphenyl)-5,7-dihydroxychromen-4-one; (c) α; (d) Isoflavonoids; (e) Polyphenols.

[102] (a) daidzein; (b) 7-hydroxy-3-(4-hydroxyphenyl)chromen-4-one; (c) α; (d) Isoflavonoids; (e) Polyphenols.

[103] (a) 6-hydroxydaidzein; (b) 6,7-dihydroxy-3-(4-hydroxyphenyl)chromen-4-one; (c) α; (d) Isoflavonoids; (e) Polyphenols.

[104] (a) biochanin A; (b) 5,7-dihydroxy-3-(4-methoxyphenyl)chromen-4-one; (c) α; (d) Isoflavonoids; (e) Polyphenols.

[105] (a) formononetin; (b) 7-hydroxy-3-(4-methoxyphenyl)chromen-4-one; (c) α; (d) Isoflavonoids; (e) Polyphenols.

[106] (a) tectoridin; (b) 5-hydroxy-3-(4-hydroxyphenyl)-6-methoxy-7-[(2S,3R,4S,5S,6R)-3,4,5-trihydroxy-6-(hydroxymethyl)oxan-2-yl]oxychromen-4-one; (c) α; (d) Isoflavonoids; (e) Polyphenols.

[107] (a) Bilobetin; (b) 8-[5-(5,7-dihydroxy-4-oxochromen-2-yl)-2-methoxyphenyl]-5,7-dihydroxy-2-(4-hydroxyphenyl)chromen-4-one; (c) α; (d) Biflavonoids; (e) Polyphenols.

[108] (a) picrasidine C; (b) 4-(4,8-dimethoxy-9H-pyrido[3,4-b]indol-1-yl)-2-methoxy-1-(9H-pyrido[3,4-b]indol-1-yl)butan-1-one; (c) α; (d) Alkaloids; (e) Alkaloids.

[109] (a) berberine; (b) 9,10-Dimethoxy-5,6-dihydro[1,3]dioxolo[4,5-g]isoquinolino[3,2-a]isoquinolin-7-ium; (c) α; (d) Alkaloids; (e) Alkaloids.

[110] (a) oxymatrine; (b) (7aS,13aR,13bR,13cS)dodecahydro-1H,5H,10H-dipyrido[2,1-f:3',2',1'-ij][1,6]naphthyridin-10-one 4-oxide; (c) α; (d) Alkaloids; (e) Alkaloids.

[111] (a) capsaicin; (b) (E)-N-[(4-hydroxy-3-methoxyphenyl)methyl]-8-methylnon-6-enamide; (c) α; (d) Alkaloids; (e) Alkaloids.

[112] (a) S26948; (b) dimethyl 2-[[4-[2-(6-benzoyl-2-oxo-1,3-benzothiazol-3-yl)ethoxy]phenyl]methyl]propanedioate; (c) γ; (d) -; (e) -.

[113] (a) Efatutazone Hydrochloride (RS5444); (b) 5-[[4-[[6-(4-amino-3,5-dimethylphenoxy)-1-methylbenzimidazol-2-yl]methoxy]phenyl]methyl]-1,3-thiazolidine-2,4-dione;dihydrochloride; (c) γ; (d) -; (e) -.

[114] (a) Edaglitazone; (b) 5-[[4-[2-(5-methyl-2-phenyl-1,3-oxazol-4-yl)ethoxy]-1-benzothiophen-7-yl]methyl]-1,3-thiazolidine-2,4-dione; (c) γ; (d) Glitazone; (e) -.

[115] (a) GW 1929 hydrochloride; (b) (2S)-2-(2-benzoylanilino)-3-[4-[2-[methyl(pyridin-2-yl)amino]ethoxy]phenyl]propanoic acid;hydrochloride; (c) γ; (d) -; (e) -.

[116] (a) LG 100754; (b) (2E,4E,6Z)-3-methyl-7-(5,5,8,8-tetramethyl-3-propoxy-6,7-dihydronaphthalen-2-yl)octa-2,4,6-trienoic acid; (c) γ; (d) -; (e) -.

[117] (a) CP 775146; (b) 2-methyl-2-[3-[(3S)-1-[2-(4-propan-2-ylphenyl)acetyl]piperidin-3-yl]phenoxy]propanoic acid; (c) α; (d) -; (e) -.

[118] (a) IVA337; (b) 4-[1-(1,3-benzothiazol-6-ylsulfonyl)-5-chloroindol-2-yl]butanoic acid; (c) pan-PPAR; (d) ; (e) -.

[119] (a) Etofylline clofibrate (Theofibrate); (b) 2-(1,3-dimethyl-2,6-dioxopurin-7-yl)ethyl 2-(4-chlorophenoxy)-2-methylpropanoate; (c) α; (d) -; (e) fibrate.

[120] (a) Simfibrate; (b) 3-[2-(4-chlorophenoxy)-2-methylpropanoyl]oxypropyl 2-(4-chlorophenoxy)-2-methylpropanoate; (c) α; (d) -; (e) fibrate.

[121] (a) Ronifibrate; (b) 3-[2-(4-chlorophenoxy)-2-methylpropanoyl]oxypropyl pyridine-3-carboxylate; (c) α; (d) -; (e) fibrate.

[122] (a) Etofibrate; (b) 2-[2-(4-chlorophenoxy)-2-methylpropanoyl]oxyethyl pyridine-3-carboxylate; (c) α; (d) -; (e) fibrate.

[123] (a) Clofibride; (b) [4-(dimethylamino)-4-oxobutyl] 2-(4-chlorophenoxy)-2-methylpropanoate; (c) α; (d) -; (e) fibrate.

[124] (a) Clinofibrate; (b) 2-[4-[1-[4-(2-carboxybutan-2-yloxy)phenyl]cyclohexyl]phenoxy]-2-methylbutanoic acid; (c) α; (d) -; (e) fibrate.

[125] (a) Halofenate; (b) 2-acetamidoethyl 2-(4-chlorophenyl)-2-[3-(trifluoromethyl)phenoxy]acetate; (c) α; (d) -; (e) fibrate.

[126] (a) Lifibrate; (b) (1-methylpiperidin-4-yl) 2,2-bis(4-chlorophenoxy)acetate; (c) α; (d) -; (e) fibrate.

[127] (a) Lifibrol; (b) 4-[4-(4-tert-butylphenyl)-2-hydroxybutoxy]benzoic acid; (c) α; (d) -; (e) fibrate.

[128] (a) Nafenopin; (b) 2-methyl-2-[4-(1,2,3,4-tetrahydronaphthalen-1-yl)phenoxy]propanoic acid; (c) α; (d) -; (e) fibrate.

[129] (a) Tibric acid; (b) 2-chloro-5-[(3S,5R)-3,5-dimethylpiperidin-1-yl]sulfonylbenzoic acid; (c) $\alpha$; (d) -; (e) fibrate.

[130] (a) Treloxinate; (b) methyl 3,7-dichloro-5H-benzo[d][1,3]bcnzodioxocine-11-carboxylate; (c) $\alpha$; (d) -; (e) fibrate.

[131] (a) Binifibrate; (b) [2-[2-(4-chlorophenoxy)-2-methylpropanoyl]oxy-3-(pyridine-3-carbonyloxy)propyl] pyridine-3-carboxylate; (c) $\alpha$; (d) -; (e) fibrate.

[132] (a) Clofibric acid; (b) 2-(4-chlorophenoxy)-2-methylpropanoic acid; (c) $\alpha$; (d) -; (e) fibrate.

[133] (a) Darglitazone sodium; (b) sodium;5-[[4-[3-(5-methyl-2-phenyl-1,3-oxazol-4-yl)propanoyl]phenyl]methyl]-1,3-thiazolidin-3-ide-2,4-dione; (c) $\alpha$; (d) -; (e) Thiazolidinedione.

[134] (a) Edaglitazone sodium; (b) sodium;5-[[4-[2-(5-methyl-2-phenyl-1,3-oxazol-4-yl)ethoxy]-1-benzothiophen-7-yl]methyl]-1,3-thiazolidin-3-ide-2,4-dione; (c) $\alpha$; (d) -; (e) Thiazolidinedione.

[135] (a) Englitazone sodium; (b) sodium;5-[[(2R)-2-benzyl-3,4-dihydro-2H-chromen-6-yl]methyl]-1,3-thiazolidin-3-ide-2,4-dione; (c) $\alpha$; (d) -; (e) Thiazolidinedione.

[136] (a) Netoglitazone; (b) 5-[[6-[(2-fluorophenyl)methoxy]naphthalen-2-yl]methyl] -1,3-thiazolidine-2,4-dione; (c) $\alpha$; (d) -; (e) Thiazolidinedione.

[137] (a) Rivoglitazone; (b) 5-[[4-[(6-methoxy-1-methylbenzimidazol-2-yl)methoxy]phenyl]methyl]-1,3-thiazolidine-2,4-dione; (c) $\alpha$; (d) -; (e) Thiazolidinedione.

[138] (a) Reglitazar; (b) 4-[[4-[2-(5-methyl-2-phenyl-1,3-oxazol-4-yl)ethoxy]phenyl]methyl]-1,2-oxazolidine-3,5-dione; (c) $\alpha$; (d) -; (e) Non-thiazolidinedione type PPAR agonist.

[139] (a) Peliglitazar; (b) 2-[(4-methoxyphenoxy)carbonyl-[(1S)-1-[4-[2-(5-methyl-2-phenyl-1,3-oxazol-4-yl)ethoxy]phenyl]ethyl]amino]acetic acid; (c) $\alpha$; (d) -; (e) Non-thiazolidinedione type PPAR agonist.

[140] (a) Sodelglitazar; (b) 2-[4-[[2-[2-fluoro-4-(trifluoromethyl)phenyl]-4-methyl-1,3-thiazol-5-yl]methylsulfanyl]-2-methylphenoxy]-2-methylpropanoic acid; (c) $\alpha$; (d) -; (e) Non-thiazolidinedione type PPAR agonist.

[141] (a) Naveglitazar; (b) (2S)-2-methoxy-3-[4-[3-(4-phenoxyphenoxy)propoxy]phenyl]propanoic acid; (c) $\alpha$; (d) -; (e) Non-thiazolidinedione type PPAR agonist.

[142] (a) Indeglitazar; (b) 3-[5-methoxy-1-(4-methoxyphenyl)sulfonylindol-3-yl]propanoic acid; (c) $\alpha$; (d) -; (e) Non-thiazolidinedione type PPAR agonist.

[143] (a) Arhalofenate; (b) 2-acetamidoethyl (2R)-2-(4-chlorophenyl)-2-[3-(trifluoromethyl)phenoxy]acetate; (c) $\alpha$; (d) -; (e) Non-thiazolidinedione type PPAR agonist.

[144] (a) 10-Nitrooleate; (b) (9E)-10-Nitro-9-octadecenoic acid; (c) $\gamma$; (d) -; (e) -.

[145] (a) 13(S)-HODE; (b) (9Z,11E)-13-Hydroxy-9,11-octadecadienoic acid; (c) $\gamma$; (d) -; (e) -.

[146] (a) 20-carboxy Arachidonic acid; (b) (5E,8E,11E,14E)-5,8,11,14-Icosatetraenedioic acid; (c) $\gamma$; (d) -; (e) -.

[147] (a) 9-Nitrooleate; (b) (9E)-9-Nitro-9-octadecenoic acid; (c) $\gamma$; (d) -; (e) -.

[148] (a) Azelaoyl-PAF; (b) (2-{[(2R)-2-[(8-carboxyoctanoyl)oxy]-3-(hexadecyloxy)propyl phosphonato]oxy}ethyl)trimethylazanium; (c) $\gamma$; (d) -; (e) -.

[149] (a) Carnosic acid; (b) (4aR,10aS)-5,6-dihydroxy-1,1-dimethyl-7-propan-2-yl-2,3,4,9,10,10a-hexahydrophenanthrene-4a-carboxylic acid; (c) $\gamma$; (d) -; (e)

[150] (a) CAY10506; (b) N-[2-[4-[(2,4-dioxo-1,3-thiazolidin-5-yl)methyl]phenoxy]ethyl]-5-(dithiolan-3-yl)pentanamide; (c) $\gamma$; (d) -; (e) -.

[151] (a) CAY10599; (b) 2-[(1-{3-[4-(4-Biphenylylcarbonyl)-2-propylphenoxy]propyl}-1,2,3,4-tetrahydro-5-quinolinyl)oxy]-2-methylpropanoic acid; (c) $\gamma$; (d) -; (e) -.

[152] (a) CAY15073; (b) 2-[6-[3-(6-benzoyl-1-propylnaphthalen-2-yl)oxypropoxy]indol-1-yl]acetic acid; (c) $\gamma$; (d) -; (e) -.

[153] (a) Conjugated Linoleic Acid; (b) (9Z,11E)-octadeca-9,11-dienoicacid; (c) $\gamma$; (d) -; (e) -.

[154] (a) DRF2519; (b) 5-[[4-[2-(4-oxo-2H-1,3-benzoxazin-3-yl)ethoxy]phenyl]methyl]-1,3-thiazolidine-2,4-dione; (c) $\gamma$; (d) -; (e) -.

[155] (a) ETYA; (b) icosa-5,8,11,14-tetraynoic acid; (c) $\gamma$; (d) -; (e) -.

[156] (a) glitazone Hydrochloride; (b) 5-{4-[2-(5-Ethyl-2-pyridinyl)ethoxy]benzyl}-1,3-thiazolidine-2,4-dione hydrochloride; (c) $\gamma$; (d) -; (e) -.

[157] (a) GQ-16; (b) (5Z)-5-(5-Bromo-2-methoxybenzylidene)-3-(4-methylbenzyl)-1,3-thiazolidine-2,4-dione; (c) $\gamma$; (d) -; (e) -.

[158] (a) GW6471; (b) N-[(2S)-3-[4-[2-(5-methyl-2-phenyl-1,3-oxazol-4-yl)ethoxy]phenyl]-2-[[(Z)-4-oxo-4-[4-(trifluoromethyl)phenyl]but-2-en-2-yl]amino]propyl]propanamide; (c) $\alpha$; (d) -; (e) -.

[159] (a) LY171883; (b) 1-{2-Hydroxy-3-propyl-4-[4-(1H-tetrazol-5-yl)butoxy]phenyl}ethanone; (c) $\gamma$; (d) -; (e) -.

[160] (a) Methyl-8-hydroxy-8-(2-pentyl-oxyphenyl)-oct-5-ynoate; (b) Methyl 8-hydroxy-8-[2-(pentyloxy)phenyl]-5-octynoate; (c) $\gamma$; (d) -; (e) -.

[161] (a) PAz-PC; (b) [(2R)-2-(8-carboxyoctanoyloxy)-3-hexadecanoyloxypropyl] 2-(trimethylazaniumyl)ethyl phosphate; (c) $\gamma$; (d) -; e)-.

[162] (a) PGPC; (b) [(2R)-2-(4-carboxybutanoyloxy)-3-hexadecanoyloxypropyl] 2-(trimethylazaniumyl)ethyl phosphate; (c) $\gamma$; (d) -; (e) -.

[163] (a) Sulfasalazine; (b) 2-hydroxy-5-[(E)-2-{4-[(pyridin-2-yl)sulfamoyl]phenyl]diazen-1-yl]benzoic acid; (c) γ; (d) -; (e) -.

In the present embodiment, the PPAR activator is more preferably bezafibrate, fenofibrate, ciprofibrate, clofibrate, rosiglitazone, pioglitazone, gemfibrozil, or etofibrate, and is even more preferably rosiglitazone, ciprofibrate, or gemfibrozil.

Note that any of the above-listed PPAR activators may be used alone or two or more of them may be used in combination.

In a case where there are one or more geometric isomers and/or optical isomers of the PPAR activator in accordance with the present embodiment, such one or more isomers are also included within the scope of the PPAR activator in accordance with the present embodiment.

In a case where there are one or more proton tautomers of the PPAR activator in accordance with the present embodiment, such one or more tautomers (keto tautomer, enol tautomer) are also included within the scope of the PPAR activator in accordance with the present embodiment.

In a case where there are one or more hydrates and/or one or more solvates of the PPAR activator in accordance with the present embodiment, such one or more hydrates and/or one or more solvates are also included within the scope of the PPAR activator in accordance with the present embodiment.

In a case where there are one or more crystal polymorphs and/or one or more crystal polymorph groups (crystal polymorph systems) for the PPAR activator in accordance with the present embodiment, such one or more crystal polymorphs and/or one or more crystal polymorph groups (crystal polymorph systems) are also included within the scope of the PPAR activator in accordance with the present embodiment. As used herein, the term "crystal polymorph groups (crystal polymorph system)" is, in a case where a crystal form changes variously depending on the conditions and/or states in which crystals are, for example, produced, crystallized, or preserved (note that those "states" also include formulated states), a generic term for "crystalline forms appearing in a specific stage and/or crystalline forms appearing in every stage".

In one embodiment, a derivative of or a salt of any of the foregoing substances may be used as the PPAR activator. Use of a derivative of or a salt of any of the foregoing substances provides the following effects, for example: (1) the preventive effect and/or therapeutic effect on cataract can be increased; (2) safety of a substance to the human body can be increased; and (3) a formulation can be prepared using a substance that is easy to handle.

In this specification, the term "derivative" of a specific compound refers to any of compounds derived from that specific compound as a result of substitution of a part of the molecule of the specific compound with some other functional group or some other atom.

Examples of such other functional group include alkyl groups, alkoxy groups, alkylthio groups, aryl groups, aryloxy groups, arylthio groups, arylalkyl groups, arylalkoxy groups, arylalkylthio groups, arylalkenyl groups, arylalkyny groups, allyl group, amino groups, substituted amino groups, silyl groups, substituted silyl groups, silyloxy group, substituted silyloxy groups, arylsulfonyloxy groups, alkylsulfonyloxy groups, nitro group, and the like. Examples of such other atom include carbon atom, hydrogen atom, oxygen atom, nitrogen atom, sulfur atom, phosphorus atom, halogen atoms, and the like.

In this specification, the term "salt" refers to a salt that is physiologically acceptable to a subject. Examples of the salt include alkali metal salts (such as lithium salt, sodium salt, potassium salt), alkaline earth metal salts (such as calcium salt, magnesium salt), metal salts (such as salts of any of the foregoing substances and iron, zinc, or the like), salts of any of the foregoing substances and ammonia, quaternary ammonium salts (such as salts of any of the foregoing substances and methyl bromide, methyl iodide, or the like), salts of any of the foregoing substances and halide ion (such as salts of any of the foregoing substances and bromide ion, chloride ion, iodide ion, or the like), organic basic salts (such as trimethylamine salt, triethylamine salt, triethylenediamine salt, 2-aminoethanol salt, pyridine salt, picoline salt, dicyclohexylamine salt, N,N'-dibenzylethylenediamine salt), organic acid salts (such as acetate, maleate, fumarate, succinate, citrate, adipate, gluconate, glucuronate, terephthalate, lactate, hippurate, oleate, pamoate, stearate, tannate, tartrate, methanesulfonate, benzenesulfonate, formate, toluenesulfonate, trifluoroacetate), and inorganic acid salts (such as hydrochloride, hydrobromide, nitrate, sulfate, phosphate).

The foregoing PPAR activator can be produced in accordance with a known method. A commercially available PPAR activator can be used as the foregoing PPAR activator.

[4. Agent for prevention and/or therapeutic treatment of cataract]

An agent for prevention and/or therapeutic treatment of cataract in accordance with an embodiment of the present invention contains the foregoing PPAR activator as an active ingredient. The following description will discuss the agent for prevention and/or therapeutic treatment of cataract in accordance with the present embodiment.

[4-1. Administration route and dosage form]

An agent for prevention and/or therapeutic treatment of cataract in accordance with an embodiment of the present invention is administered to a subject. In one embodiment, the subject is a human. In another embodiment, the subject is an animal other than human. Examples of the animal other than human include non-human mammals (such as cattle, swine, sheep, goats, horses, dogs, cats, rabbits, mice, rats).

The agent for prevention and/or therapeutic treatment of cataract in accordance with an embodiment of the present invention can be administered to a subject via any pharmaceutically acceptable route of administration. Examples of the route of administration include peroral administration, parenteral administration, transdermal administration, transmucosal administration, and intravenous administration. Accordingly, the dosage form of the agent for prevention and/or therapeutic treatment of cataract can be an internal medicine (agent for internal use), external medicine (agent for external administration), injection, suppository, inhalant, ophthalmic agent, or the like.

Among the routes of administration listed above, parenteral administration and transdermal administration are preferred, administration by instillation to an eye, intra-conjunctival sac administration, intracameral administration, intravitreal administration, subconjunctival administration, transdermal administration, and sub-Tenon's capsule administration are more preferred, and administration by instillation to an eye is even more preferred. Accordingly, among the dosage forms listed above, ophthalmic agent, ointment, cream, gel, transdermal formulation, adhesive skin patch, and injection are preferred, and ophthalmic agent is more preferred. The ophthalmic agent may be in the form of water-based eye drops obtained by dissolving an agent in water or in the form of a suspension or emulsion.

The reasons why ophthalmic agent is preferred are, for example, as follows: the route through which the active ingredient is delivered to the crystalline lens is short; other organs are less likely to be stimulated; almost no invasion is caused; systemic effect is small (application of pressure on lacrimal point after instillation further reduces the systemic effect); administration is easy; repetitive administration is available; a patient is able to self-care; and the like. According to the above arrangement, the preventive effect or therapeutic effect on cataract can be further accelerated and further increased.

[4-2. Components]

(Active ingredient)

The agent for prevention and/or therapeutic treatment of cataract in accordance with an embodiment of the present invention contains a PPAR activator as an active ingredient. In this specification, the term "active ingredient" refers to a substance that is capable of providing a preventive effect on one or more symptoms and/or a therapeutic effect on one or more symptoms.

The concentration of the PPAR activator contained in the agent for prevention and/or therapeutic treatment of cataract in accordance with an embodiment of the present invention can vary depending on the type of PPAR activator, route of administration of the agent for prevention and/or therapeutic treatment, dosage form of the agent for prevention and/or therapeutic treatment, and/or the like.

In one example, the concentration of the PPAR activator in the agent for prevention and/or therapeutic treatment is, for example, 0.001 $\mu$M or more, 0.002 $\mu$M or more, 0.005 $\mu$M or more, 0.01 $\mu$M or more, 0.02 $\mu$M or more, 0.05 $\mu$M or more, 0.1 $\mu$M or more, 0.2 $\mu$M or more, 0.5 $\mu$M or more, 1 $\mu$M or more, 2 $\mu$M or more, 3 $\mu$M or more, 4 $\mu$M or more, 5 $\mu$M or more, 6 $\mu$M or more, 7 $\mu$M or more, 8 $\mu$M or more, 9 $\mu$M or more, 10 $\mu$M or more, 20 $\mu$M or more, 30 $\mu$M or more, 40 $\mu$M or more, 50 $\mu$M or more, 60 $\mu$M or more, 70 $\mu$M or more, 80 $\mu$M or more, 90 $\mu$M or more, 100 $\mu$M or more, 200 $\mu$M or more, 300 $\mu$M or more, 400 $\mu$M or more, 500 $\mu$M or more, 600 $\mu$M or more, 700 $\mu$M or more, 800 $\mu$M or more, 900 $\mu$M or more, or 1000 $\mu$M or more.

In one example, the concentration of the PPAR activator in the agent for prevention and/or therapeutic treatment is, for example, 0.01 $\mu$M or less, 0.02 $\mu$M or less, 0.05 $\mu$M or less, 0.1 $\mu$M or less, 0.2 $\mu$M or less, 0.5 $\mu$M or less, 1 $\mu$M or less, 2 $\mu$M or less, 3 $\mu$M or less, 4 $\mu$M or less, 5 $\mu$M or less, 6 $\mu$M or less, 7 $\mu$M or less, 8 $\mu$M or less, 9 $\mu$M or less, 10 $\mu$M or less, 20 $\mu$M or less, 30 $\mu$M or less, 40 $\mu$M or less, 50 $\mu$M or less, 60 $\mu$M or less, 70 $\mu$M or less, 80 $\mu$M or less, 90 $\mu$M or less, 100 $\mu$M or less, 200 $\mu$M or less, 300 $\mu$M or less, 400 $\mu$M or less, 500 $\mu$M or less, 600 $\mu$M or less, 700 $\mu$M or less, 800 $\mu$M or less, 900 $\mu$M or less, 1000 $\mu$M or less, 2000 $\mu$M or less, 3000 $\mu$M or less, 4000 $\mu$M or less, 5000 $\mu$M or less, 10 mM or less, 50 mM or less, 100 mM or less, or 200 mM or less.

In one example, the concentration of the PPAR activator in the agent for prevention and/or therapeutic treatment is, for example, 0.001 $\mu$M to 5000 $\mu$M, 0.001 $\mu$M to 0.01 $\mu$M, 0.002 $\mu$M to 0.02 $\mu$M, 0.005 $\mu$M to 0.05 $\mu$M, 0.01 $\mu$M to 0.1 $\mu$M, 0.02 $\mu$M to 0.2 $\mu$M, 0.05 $\mu$M to 0.5 $\mu$M, 0.1 $\mu$M to 1 $\mu$M, 0.2 $\mu$M to 2 $\mu$M, 0.3 $\mu$M to 3 $\mu$M, 0.4 $\mu$M to 4 $\mu$M, 0.5 $\mu$M to 5 $\mu$M, 0.6 $\mu$M to 6 $\mu$M, 0.7 $\mu$M to 7 $\mu$M, 0.8 $\mu$M to 8 $\mu$M, 0.9 $\mu$M to 9 $\mu$M, 1 $\mu$M to 10 $\mu$M, 2 $\mu$M to 20 $\mu$M, 3 $\mu$M to 30 $\mu$M, 4 $\mu$M to 40 $\mu$M, 5 $\mu$M to 50 $\mu$M, 6 $\mu$M to 60 $\mu$M, 7 $\mu$M to 70 $\mu$M, 8 $\mu$M to 80 $\mu$M, 9 $\mu$M to 90 $\mu$M, 10 $\mu$M to 100 $\mu$M, 20 $\mu$M to 200 $\mu$M, 30 $\mu$M to 300 $\mu$M, 40 $\mu$M to 400 $\mu$M, 50 $\mu$M to 500 $\mu$M, 60 $\mu$M to 600 $\mu$M, 70 $\mu$M to 700 $\mu$M,

80 μM to 800 μM, 90 μM to 900 μM, 100 μM to 1000 μM, 200 μM to 2000 μM, 500 μM to 5000 μM, 1 mM to 100 mM, or 1 mM to 200 mM.

Note that, in this specification, the unit "M" means the amount-of-substance (mol) of a component per 1 L liquid, and may alternatively be expressed in the unit "mol/L". For example, in a case of an ophthalmic agent, the unit "M" means the amount-of-substance (mol) of a component per 1L of the ophthalmic agent. In this specification, in a case where a substance serving as a PPAR activator is a salt, a value expressed in the unit "M" is the amount-of-substance of the salt per 1 L liquid. Similarly, in a case where a substance serving as a PPAR activator is in the form of a hydrate or solvate, a value expressed in the unit "M" is the amount-of-substance of the hydrate or solvate per 1 L liquid. The same applies to other forms of substance, unless otherwise specified herein.

In one example, the concentration of the PPAR activator in the agent for prevention and/or therapeutic treatment is, for example, preferably 0.001 to 10% (w/v), more preferably 0.005 to 5% (w/v), even more preferably 0.01 to 2% (w/v), even more preferably 0.1 to 1% (w/v). For example, in a case of an ophthalmic agent containing ciprofibrate, the concentration of ciprofibrate in the ophthalmic agent is preferably 0.001 to 10% (w/v), more preferably 0.005 to 5% (w/v), even more preferably 0.01 to 2% (w/v), even more preferably 0.1 to 1% (w/v). In a case of an ophthalmic agent containing gemfibrozil, the concentration of gemfibrozil in the ophthalmic agent is preferably 0.001 to 10% (w/v), more preferably 0.005 to 5% (w/v), even more preferably 0.01 to 2% (w/v), even more preferably 0.1 to 1% (w/v). In a case of an ophthalmic agent containing rosiglitazone, the concentration of rosiglitazone in the ophthalmic agent is preferably 0.001 to 10% (w/v), more preferably 0.005 to 5% (w/v), even more preferably 0.01 to 2% (w/v), even more preferably 0.1 to 1% (w/v).

Note that, in this specification, the unit "% (w/v)" means the mass (g) of a component contained in 100 mL liquid. For example, in a case of an ophthalmic agent, the unit "% (w/v)" means the mass (g) of a component contained in 100 mL of the ophthalmic agent. In this specification, in a case where a substance serving as a PPAR activator is a salt, a value expressed in the unit "% (w/v)" means the amount of the salt contained in 100 mL liquid. Similarly, in a case where a substance serving as a PPAR activator is in the form of a hydrate or solvate, a value expressed in the unit "% (w/v)" means the amount of the hydrate or solvate of the substance contained in 100 mL liquid. The same applies to other forms of substance, unless otherwise specified herein.

The agent for prevention and/or therapeutic treatment of cataract in accordance with an embodiment of the present invention may further contain an active ingredient other than PPAR activators or may contain a PPAR activator as the only active ingredient.

(Additive)

The agent for prevention and/or therapeutic treatment of cataract in accordance with an embodiment of the present invention may further contain one or more additives for medicaments, optionally in order to satisfy the requirements for pharmaceutical formulation. Examples of additives for medicaments include buffers, pH adjustors, tonicity agents, antiseptic agents, antioxidants, high molecular weight polymers, filler, carriers, diluents, solvents, solubilizing agents, stabilizers, bulking agents, binders, surfactants, stabilizing agents, and the like. Any of such additives may be used alone or two or more of them may be used in combination. The amount of such one or more additives may be selected appropriately.

Examples of the buffers include phosphoric acid, phosphates, boric acid, borates, citric acid, citrates, acetic acid, acetates, carbonic acid, carbonates, tartaric acid, tartrates, ε-aminocaproic acid, trometamol, and the like. Examples of the phosphates include sodium phosphate, sodium dihydrogen phosphate, disodium hydrogen phosphate, potassium phosphate, potassium dihydrogen phosphate, dipotassium hydrogen phosphate, and the like. Examples of the borates include borax, sodium borate, potassium borate, and the like. Examples of the citrates include sodium citrate, disodium citrate, trisodium citrate, and the like. Examples of the acetates include sodium acetate, potassium acetate, and the like. Examples of the carbonates include sodium carbonate, sodium hydrogen carbonate, and the like. Examples of the tartrates include sodium tartrate, potassium tartrate, and the like.

Examples of the pH adjustors include hydrochloric acid, phosphoric acid, citric acid, acetic acid, sodium hydroxide, potassium hydroxide, and the like.

Examples of the tonicity agents include ionic tonicity agents (such as sodium chloride, potassium chloride, calcium chloride, and magnesium chloride) and nonionic tonicity agents (such as glycerin, propylene glycol, trometamol, sorbitol, and mannitol).

Examples of the antiseptic agents include benzalkonium chloride, benzalkonium bromide, benzethonium chloride, sorbic acid, potassium sorbate, methyl parahydroxybenzoate, propyl parahydroxybenzoate, chlorhexidine gluconate, chlorobutanol, and the like.

Examples of the antioxidants include ascorbic acid, tocopherol, dibutylhydroxytoluene, butylated hydroxyanisole, sodium erythorbate, propyl gallate, sodium sulfite, and the like.

Examples of the high molecular weight polymers include methyl cellulose, ethyl cellulose, hydroxymethyl cellulose, hydroxyethyl cellulose, hydroxypropyl cellulose, hydroxyethyl methyl cellulose, hydroxypropyl methyl cellulose, carboxymethyl cellulose, carboxymethylcellulose sodium salt, hydroxypropyl methylcellulose acetate succinate, hydroxypropyl methylcellulose phthalate, carboxymethyl ethyl cellulose, cellulose acetate phthalate, polyvinylpyrrolidone, polyvinyl alcohol, carboxyvinyl polymer, polyethylene glycol, atelocollagen, and the like.

In particular, the agent for prevention and/or therapeutic treatment of cataract in accordance with an embodiment of the present invention preferably contains atelocollagen. According to the above arrangement, the preventive effect or therapeutic effect on cataract can be further accelerated and further increased.

The concentration of one or more additives contained in the agent for prevention and/or therapeutic treatment of cataract in accordance with an embodiment of the present invention is not particularly limited. The concentration of one or more additives in the agent for prevention and/or therapeutic treatment may be, for example, 0 $\mu$M, 0.001 $\mu$M to 5000 $\mu$M, 0.001 to 0.01 $\mu$M, 0.002 to 0.02 $\mu$M, 0.005 to 0.05 $\mu$M, 0.01 to 0.1 $\mu$M, 0.02 to 0.2 $\mu$M, 0.05 to 0.5 $\mu$M, 0.1 to 1 $\mu$M, 0.2 to 2 $\mu$M, 0.3 to 3 $\mu$M, 0.4 to 4 $\mu$M, 0.5 to 5 $\mu$M, 0.6 to 6 $\mu$M, 0.7 to 7 $\mu$M, 0.8 to 8 $\mu$M, 0.9 to 9 $\mu$M, 1 to 10 $\mu$M, 2 to 20 $\mu$M, 3 to 30 $\mu$M, 4 to 40 $\mu$M, 5 to 50 $\mu$M, 6 to 60 $\mu$M, 7 to 70 $\mu$M, 8 to 80 $\mu$M, 9 to 90 $\mu$M, 10 to 100 $\mu$M, 20 to 200 $\mu$M, 30 to 300 $\mu$M, 40 to 400 $\mu$M, 50 to 500 $\mu$M, 60 to 600 $\mu$M, 70 to 700 $\mu$M, 80 to 800 $\mu$M, 90 to 900 $\mu$M, 100 to 1000 $\mu$M, 200 to 2000 $\mu$M, or 500 to 5000 $\mu$M.

[4-3. Formulation and prescription]

With use of the foregoing PPAR activator and the foregoing one or more additives as source materials, it is possible to produce an agent for prevention and/or therapeutic treatment of cataract in accordance with an embodiment of the present invention by a known method.

In a case where the agent for prevention and/or therapeutic treatment of cataract in accordance with an embodiment of the present invention is, for example, an ophthalmic agent, the components contained in the ophthalmic agent may be totally dissolved or may be partially suspended. It is preferable that the components contained in the ophthalmic agent are totally dissolved, and that the ophthalmic agent is in the form of liquid.

When the agent for prevention and/or therapeutic treatment of cataract in accordance with an embodiment of the present invention is administered (e.g., administered as an ophthalmic agent), there is no limitation on dosing interval, provided that a desired effect is achieved. The dosing interval is, for example, once every hour to once every six months, preferably once every hour, once every two hours, once every three hours, once every six hours, once every twelve hours, once a day, twice a day, three times a day, four times a day, five times a day, six times a day, once every two days, once every three days, once every four days, once every five days, once every six days, once a week, once every two weeks, once every three weeks, once a month, once every two months, once every three months, once every four months, once every five months, once every six months, more preferably at least once a day, at least once every two days, at least once every three days, at least once every four days, at least once every five days, at least once every six days, at least once a week.

The agent for prevention and/or therapeutic treatment of cataract in accordance with an embodiment of the present invention may be prescribed in combination with some other agent for prevention or therapeutic treatment of cataract and/or some other disease.

In one aspect, the present invention is directed to a pharmaceutical composition for prevention and/or therapeutic treatment of cataract, the pharmaceutical composition containing a PPAR activator as an active ingredient.

Another aspect of the present invention is directed to use of a PPAR activator for production of a medicament for prevention and/or therapeutic treatment of cataract (agent for prevention and/or therapeutic treatment of cataract).

A further aspect of the present invention is directed to a method for prevention and/or therapeutic treatment of cataract, the method including using a PPAR activator.

Still a further aspect of the present invention is directed to a method for prevention and/or therapeutic treatment of cataract, the method including the step of administering, to a subject, an agent for prevention and/or therapeutic treatment of cataract containing a PPAR activator as an active ingredient.

Still a further aspect of the present invention is directed to a PPAR activator for use in prevention and/or therapeutic treatment of cataract.

In still a further aspect of the present invention, the scope of the present invention also includes (i) an ophthalmic agent containing ciprofibrate as an active ingredient (e.g., the concentration of ciprofibrate is 0.001 to 10% (w/v)), (ii) an ophthalmic agent containing gemfibrozil as an active ingredient (e.g., the concentration of gemfibrozil is 0.001 to 10% (w/v)), and (iii) an ophthalmic agent containing rosiglitazone as an active ingredient (e.g., the concentration of rosiglitazone is 0.001 to 10% (w/v)). Such ophthalmic agents may be used for prevention and/or therapeutic treatment of cataract or may be used for some other purpose.

In these aspects, various conditions including the type of PPAR activator, how the PPAR activator is administered, administration route of the PPAR activator, subject who receives administration of the PPAR activator, dosage of the PPAR activator, and the like can be the same as those concerning the "agent for prevention and/or therapeutic treatment of cataract".

Examples

[Example 1: ex vivo study]

In Example 1, effects of each agent were tested with use of ex-vivo diabetic cataract models using galactose-containing media.

The crystalline lenses were isolated from the right and left eyeballs of a 6-week-old SD rat (available from Sankyo Labo Service Corporation).

The isolated crystalline lenses were cultured with use of a medium obtained by adding 30 mM of galactose to an M199 medium (available from SIGMA). After that, the crystalline lenses were cultured with use of a medium obtained by adding 30 mM of galactose and 800 $\mu$M of a PPAR activator (specifically, bezafibrate or fenofibrate) to an M199 medium.

Specific points in time at which observation was carried out are as follows. Note that the crystalline lenses were cultured with use of an incubator, the internal temperature of which was maintained at 37°C.

First, 3 days after the start of the culture of the crystalline lenses using the medium obtained by adding 30 mM of galactose to an M199 medium (available from SIGMA), the crystalline lenses were removed from the medium and observed by microscopy under a SZX12 microscope (available from Olympas).

After the microscopy, a PPAR activator was added to the medium, and the culture of the crystalline lenses was restarted. 3 days after the restart of the culture, the crystalline lenses were removed from the medium and observed by microscopy under the SZX12 microscope (available from Olympas).

Fig. 2 shows the results obtained by microscopy. Note that (A) of Fig. 2 shows the results of the tests on bezafibrate, whereas (B) of Fig. 2 shows the results of the tests on fenofibrate. Also note that, in (A) and (B) of Fig. 2, each of the legends "Day 3" indicates an image of a crystalline lens obtained 3 days after the start of the culture thereof using the medium obtained by adding 30 mM of galactose to an M199 medium (available from SIGMA), whereas each of the legends "Day 6" indicates an image of the crystalline lens obtained 3 days after the restart of the culture.

As is apparent from (A) and (B) of Fig. 2, the isolated crystalline lens has experienced progress of opacity of the cortex from the start of the culture to 3 days after the culture. On the contrary, the crystalline lens, which had been cultured again using a medium having a PPAR activator added thereto, had a decreased degree of opacity of the cortex 3 days after the restart of the culture.

These test results suggest that bezafibrate and fenofibrate, which are PPAR activators, have the effect of therapeutic treatment of cataract after its development.

[Example 2: Quantitative ex vivo study]

In Example 2, effects of each agent were tested with use of ex-vivo diabetic cataract models using galactose-containing media, in a similar manner to Example 1.

The crystalline lenses were isolated from the right and left eyeballs of a 6-week-old SD rat (available from Sankyo Labo Service Corporation).

The isolated crystalline lenses were cultured with use of a medium obtained by adding 30 mM of galactose to an M199 medium (available from SIGMA). After that, the crystalline lenses were cultured with use of a medium obtained by adding 30 mM of galactose and a PPAR activator to an M199 medium.

Specific points in time at which observation was carried out are as follows. Note that the crystalline lenses were cultured with use of an incubator, the internal temperature of which was maintained at 37°C.

First, 3 days after the start of the culture of the crystalline lenses using the medium obtained by adding 30 mM of galactose to an M199 medium (available from SIGMA), the crystalline lenses were removed from the medium and observed by microscopy under a microscope SZX12 (available from Olympas).

After the microscopy, a PPAR activator was added to the medium, and the culture of the crystalline lenses was restarted. 3 days after the restart of the culture, the crystalline lenses were removed from the medium and observed again by microscopy under the microscope SZX12 (available from Olympas). Note that, for the purpose of clarifying the effects of the PPAR activator, control crystalline lenses were cultured again using a medium having no PPAR activators added thereto.

The images obtained by the microscopy were imported into image processing software (Image J), and the number of pixels was analyzed. In this way, the total area of the crystalline lens and the area of the opaque area were calculated. The percentage of opacity of the crystalline lens was calculated using the following equation.

$$\text{Percentage of opacity of crystalline lens (\%)} = 100 \times \{(\text{area of opaque area of crystalline lens}) / (\text{total area of crystalline lens})\}$$

The test results are shown in Table 1. Table 1 shows (i) how the percentage of opacity of the crystalline lens changed when the type of PPAR activator and its concentration were changed and (ii) how the percentage of opacity of the crystalline lens changed in a case where the crystalline lens was cultured again using a medium with no PPAR activators added thereto (control).

[Table 1]

| PPAR activator | Concentration | Percentage of opacity of crystalline lens | |
|---|---|---|---|
| | | Day 3 | Day 6 |
| Ciprofibrate | 25 μM | 17.5% | 15.7% |
| | 100 μM | 16.7% | 2.0% |
| Clofibrate | 25 μM | 9.7% | 27.3% |
| | 100 μM | 7.6% | 5.7% |
| Etofibrate | 25 μM | 16.8% | 25.2% |
| | 100 μM | 11.4% | 3.2% |
| Gemfibrozil | 0.8 μM | 15.4% | 10.3% |
| | 25 μM | 13.8% | 8.2% |
| Rosiglitazone | 20 μM | 22.8% | 20.9% |
| | 80 μM | 15.6% | 2.2% |
| Bezafibrate | 200 μM | 27.1% | 28.0% |
| (Reference) Without addition of P PAR activator (First run) | - | 16.3% | 25.0% |
| (Reference) Without addition of P PAR activator (Second run) | - | 15.8% | 23.5% |

The results shown in Table 1 demonstrate that, among PPAR activators, especially ciprofibrate, gemfibrozil, and rosiglitazone have the effect of reducing the percentage of opacity of the crystalline lens even when used at low concentration.

[Example 3: in vivo study]

In Example 3, effects of each agent were tested with use of in-vivo diabetic cataract models raised on galactose feed.

For the purpose of inducing cataract, a 3-week-old SD rat (available from Sankyo Labo Service Corporation) was raised for 1 week on feed containing 25% galactose. Concurrently, during the feeding period, ophthalmic agents were administered by instillation to both eyes of the rat every day (four times a day). The components of the ophthalmic agents were as follows: dimethyl sulfoxide (DMSO; administered as a control) for the right eye; and DMSO containing a PPAR activator for the left eye. On the last day of the feeding period, the crystalline lenses were isolated from the right and left eyes of the rat, and observed by microscopy.

The type of PPAR activator administered to the left eye and its concentration in Example 3 are as follows.

| | |
|---|---|
| Ciprofibrate | 200 mM |
| Etofibrate | 140 mM |
| Gemfibrozil | 200 mM |

(continued)

| Pioglitazone | 200 mM |
| Rosiglitazone | 200 mM |

The total area of the crystalline lens, the area of the opaque area of the crystalline lens, and the percentage of opacity of the crystalline lens were calculated in the same manner as described in Example 2. The test results are shown in Table 2.

[Table 2]

| PPAR activator | Percentage of opacity of crystalline lens | |
| --- | --- | --- |
| | Left eye | Right eye |
| Ciprofibrate | 30.2% | 48.6% |
| Etofibrate | 42.8% | 43.8% |
| Gemfibrozil | 40.4% | 53.7% |
| Pioglitazone | 44.2% | 40.9% |
| Rosiglitazone | 30.6% | 48.1% |

The results shown in Table 2 demonstrate that, among PPAR activators, especially ciprofibrate, gemfibrozil and rosiglitazone have the effect of reducing the area of the opaque area of the crystalline lens in vivo. On the contrary, pioglitazone did not show the effect of reducing the area of the opaque area of the crystalline lens in vivo.

Industrial Applicability

The present invention is usable for an agent for prevention and/or therapeutic treatment of cataract.

**Claims**

1. An agent for prevention and/or therapeutic treatment of cataract, comprising a PPAR activator as an active ingredient.

2. The agent as set forth in claim 1, wherein the PPAR activator is at least one selected from the group consisting of fibrates, thiazolidines, glitazones, and glitazars.

3. The agent as set forth in claim 2, wherein the fibrates include ciprofibrate and gemfibrozil.

4. The agent as set forth in claim 2, wherein the thiazolidines include rosiglitazone.

5. The agent as set forth in any one of claims 1 to 4, wherein the agent is administered by instillation to an eye.

6. The agent as set forth in any one of claims 1 to 5, wherein the agent is an ophthalmic agent.

7. The agent as set forth in any one of claims 1 to 6, wherein the cataract is diabetic cataract.

8. A pharmaceutical composition for prevention and/or therapeutic treatment of cataract, comprising a PPAR activator as an active ingredient.

9. The pharmaceutical composition as set forth in claim 8, wherein the PPAR activator is at least one selected from the group consisting of fibrates, thiazolidines, glitazones, and glitazars.

10. The pharmaceutical composition as set forth in claim 9, wherein the fibrates include ciprofibrate and gemfibrozil.

11. The pharmaceutical composition as set forth in claim 9, wherein the thiazolidines include rosiglitazone.

12. The pharmaceutical composition as set forth in any one of claims 8 to 11, wherein the pharmaceutical composition is administered by instillation to an eye.

13. The pharmaceutical composition as set forth in any one of claims 8 to 12, wherein the pharmaceutical composition is an ophthalmic agent.

14. The pharmaceutical composition as set forth in any one of claims 8 to 13, wherein the cataract is diabetic cataract.

15. Use of a PPAR activator for production of a medicament for prevention and/or therapeutic treatment of cataract.

16. The use as set forth in claim 15, wherein the PPAR activator is at least one selected from the group consisting of fibrates, thiazolidines, glitazones, and glitazars.

17. The use as set forth in claim 16, wherein the fibrates include ciprofibrate and gemfibrozil.

18. The use as set forth in claim 16, wherein the thiazolidines include rosiglitazone.

19. The use as set forth in any one of claims 15 to 18, wherein the medicament is administered by instillation to an eye.

20. The use as set forth in any one of claims 15 to 19, wherein the medicament is an ophthalmic agent.

21. The use as set forth in any one of claims 15 to 20, wherein the cataract is diabetic cataract.

22. An ophthalmic agent comprising ciprofibrate as an active ingredient.

23. An ophthalmic agent comprising gemfibrozil as an active ingredient.

24. An ophthalmic agent comprising rosiglitazone as an active ingredient.

25. The ophthalmic agent as set forth in any one of claims 22 to 24, wherein the ophthalmic agent is for prevention and/or therapeutic treatment of cataract.

26. The ophthalmic agent as set forth in claim 25, wherein the cataract is diabetic cataract.

27. The ophthalmic agent as set forth in claim 25, wherein the cataract is age-related cataract.

FIG. 1

## FIG. 2

(A) Bezafibrate (800 μM)

Day3   Day6

(B) Fenofibrate (800 μM)

Day3   Day6

| | **INTERNATIONAL SEARCH REPORT** | International application No. |
|---|---|---|
| | | PCT/JP2018/048211 |

A. CLASSIFICATION OF SUBJECT MATTER
Int.Cl. A61K45/00(2006.01)i, A61K31/192(2006.01)i, A61K31/216(2006.01)i,
A61K31/4178(2006.01)i, A61P3/10(2006.01)i, A61P27/12(2006.01)i,
A61P43/00(2006.01)i

B. FIELDS SEARCHED

Minimum documentation searched (classification system followed by classification symbols)
Int.Cl. A61K45/00, A61K31/192, A61K31/216, A61K31/4178, A61P3/10,
A61P27/12, A61P43/00

Documentation searched other than minimum documentation to the extent that such documents are included in the fields searched
Published examined utility model applications of Japan          1922–1996
Published unexamined utility model applications of Japan        1971–2019
Registered utility model specifications of Japan               1996–2019
Published registered utility model applications of Japan       1994–2019

Electronic data base consulted during the international search (name of data base and, where practicable, search terms used)
JSTPlus/JMEDPlus/JST7580 (JDreamIII), CAplus/MEDLINE/EMBASE/BIOSIS (STN)

C. DOCUMENTS CONSIDERED TO BE RELEVANT

| Category* | Citation of document, with indication, where appropriate, of the relevant passages | Relevant to claim No. |
|---|---|---|
| X | HAN, Y. et al., "Experimental study of the therapeutic effect of rosiglitazone on cataract in type 2 diabetic model rats", Journal of Hebei Medical University, 2005, 26(2), pp. 108–111, abstract | 1–2, 4, 7–9, 11, 14–16, 18, 21, 24–27 |
| A | | 3, 5–6, 10, 12–13, 17, 19–20, 22–23 |

☒ Further documents are listed in the continuation of Box C.     ☐ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 29 January 2019 (29.01.2019) | 12 February 2019 (12.02.2019) |

| Name and mailing address of the ISA/ | Authorized officer |
|---|---|
| Japan Patent Office | |
| 3-4-3, Kasumigaseki, Chiyoda-ku, | |
| Tokyo 100-8915, Japan | Telephone No. |

Form PCT/ISA/210 (second sheet) (January 2015)

**INTERNATIONAL SEARCH REPORT**

| International application No. |
| --- |
| PCT/JP2018/048211 |

C (Continuation).   DOCUMENTS CONSIDERED TO BE RELEVANT

| Category* | Citation of document, with indication, where appropriate, of the relevant passages | Relevant to claim No. |
| --- | --- | --- |
| X<br><br>A | JP 10-139665 A (SANKYO CO., LTD.) 26 May 1998, claims 1, 9-13, paragraphs [0009], [0011], formulation example 5, examples & EP 958818 A1 claims 1, 9-13, formulation example 5, examples & WO 1998/010760 A1 | 1-2, 5-9, 12-16, 19-21<br>3-4, 10-11, 17-18, 22-27 |
| X<br><br>A | WO 00/07582 A2 (WARNER-LAMBERT COMPANY) 17 February 2000, page 22, line 23 to page 23, table (Family: none) | 1-2, 7-9, 14-16, 21<br>3-6, 10-13, 17-20, 22-27 |
| X<br><br>A | BENARDEAU, A. et al., "Effects of the dual PPAR-alpha/gamma agonist aleglitazar on glycaemic control and organ protection in the Zucker diabetic fatty rat", Diabetes Obesity and Metabolism, 2013, 15, pp. 164-174, page 165, left column, paragraph [0006] to page 165, right column, paragraph [0001], page 167, right column, paragraph [0003], fig. 3 | 1-2, 7-9, 14-16, 21<br>3-6, 10-13, 17-20, 22-27 |
| A | ALEO, M. D. et al. , "The use of explant lens culture to assess cataractogenic potential", Ann N Y Acad Sci., 2000, 919, pp. 171-187, abstract, page 178, paragraph [0002], fig. 3 | 1-27 |

Form PCT/ISA/210 (continuation of second sheet) (January 2015)

**INTERNATIONAL SEARCH REPORT**

| International application No. |
| --- |
| PCT/JP2018/048211 |

<Concerning the subject of the search>
Claim 22-24 sets forth an "eyedrop" containing ciprofibrate, gemfibrozil or rosiglitazone as an active ingredient, and since the diseases that the eyedrop is intended to treat or prevent are not specified, said eyedrop is considered to encompass eyedrops for treating or preventing every kind of disease, etc.

However, the description only discloses a pharmacological effect of suppressing the clouding of the crystalline lens in a cataract model in which ciprofibrate, gemfibrozil or rosiglitazone is isolated from rats. In addition, since it is common technical knowledge that whether each substance has a pharmacological effect on a disease differs greatly depending on the disease, it cannot be comprehended from the disclosure of the description whether the eyedrops related to claims 22-24 can be used to treat or prevent diseases other cataracts.

Thus, no basis can be found for expanding or generalizing the disclosure of the description to the scope of the invention in claims 22-24 encompassing medical uses related to the treatment or prevention of diseases other than cataracts.

Accordingly, the invention in claims 22-24 is beyond the scope disclosed in the specification, and does not comply with the requirements for support under PCT Article 6.

In addition, considering the matters mentioned above, it is not recognized that a person skilled in th art could use the eyedrop related to the invention in claims 22-24 to treat or prevent diseases other than cataracts.

Thus, the specification of the present invention is not disclosed clearly or sufficiently enough for a person skilled in the art to carry out the invention in claims 22-24, and thus does not comply with the requirements under PCT Article 5.

Accordingly, since a meaningful search cannot be carried out for the invention in claims 22-24 other than for the eyedrop for treating and/or preventing cataracts, a search was not carried out.

Form PCT/ISA/210 (extra sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **MATENSSON. J.** Glutathione ester prevents buthionine sulfoximine-induced cataracts and lens epithelial cell damage. *Proc Natl Acad Sci U S A,* 1989, vol. 86, 8727-8731

- **CIUFFI. M.** Protective Effect of Pirenoxine and U74389F on Induced Lipid Peroxidation in Mammalian Lenses. An in vitro, ex vivo and in vivo study. *EXPERIMENTAL EYE RESEARCH,* 1999, vol. 68, 347-359